# EUROPEAN PATENT SPECIFICATION

(11) **EP 0 329 098 B1**
(45) Date of publication and mention of the grant of the patent: **04.05.1994**
(21) Application number: 89102572.8
(22) Date of filing: 15.02.1989
(51) Int. Cl.: C08L 5/08, A61K 6/08

(54) **Hardenable composition**
Härtbare Zusammensetzung
Composition durcissable

(30) Priority: 16.02.1988 JP 34673/88
(43) Date of publication of application: 23.08.1989
(73) Proprietor: MATSUMOTO DENTAL COLLEGE, Shiojiri-shi, Nagano-ken (JP)
(72) Inventor: Ito, Michio, Shiojiri-shi Nagano-ken (JP)
(74) Representative: VOSSIUS & PARTNER

(56) References cited:
- EP-A- 0 298 501
- JP-A- 5 222 026
- US-A- 4 542 167
- US-A- 4 668 295
- CHEMICAL ABSTRACTS, vol. 86, no. 24, 13th June 1977, Page 297, abstract no. 176032b.; S. NAKANO et al.: "Hydraulic inorganic material with high mechanical strenght" ; & JP-A-52 022 026

## Description

The present invention relates to a hardenable composition containing chitosan. The hardenable composition of the present invention is useful, in particular, as a filling or cushioning material in dental and orthopedic fields.

Chitosan, which is a polysaccharide having amino groups, can be obtained by deacetylation of chitin which is a component of carapaces of crustaceans and insects. Chitosan has been used principally as a flocculating agent for treating water, and also in cosmetics, as a porous carrier, as an adsorbent, as a soil conditioner, and so on. These applications are based on a characteristic of chitosan as a polyamine easily forming a complex.

Japanese Patent Kokai Publication No. 22026/1977 describes a process for the production of a hardenable composition having high mechanical strength which contains chitosan and a water-hardenable inorganic material, characterized in that an acidic aqueous solution of chitosan is combined with the basic water-hardenable inorganic material to make the resulting composition basic. The process is based on the fact that solubility of chitosan is lowered under a basic condition. As the basic water-hardenable inorganic material, lime, Portland cement, alumina cement and the like are examplified. The composition is described to have a pH value of preferably not lower than 9.

On the other hand, as dental cements used for cementation of a restorative material, temporary filling, root canal filling and so on, there have been known, for example, zinc phosphate cement, zinc oxide eugenol cement and a carboxylate cement which are prepared by combining powder components with liquid components immediately before application and then hardened after 4 to 10 minutes. Such a dental cement has a pH value of 2 to 4 in the form of paste and therefore may injure dental pulp. Therefore, a neutral dental cement has been required.

US-PS-4 542 167 discloses a water-hardenable dental cement composition comprising hydroxyapatite as a main component and an inorganic powder and a hardening agent. It was found that the use of hydroxyapatite provides a dental cement having a high compression strength, a good operation adaptability and does not disintegrate due to the action of saliva. Chitosan is not mentioned in this document.

An object of the present invention is to provide a composition which can be hardened in a neutral range (pH 6 to 8).

It has been found that, a neutral, uniformly hardenable composition can be prepared by combining an acidic aqueous solution of chitosan with hydroxyapatite and zinc oxide and/or magnesium oxide.

Accordingly, the present invention provides a chitosan-containing hardenable composition in the form of a paste having a pH of 6 to 8, further comprising hydroxyapatite, and zinc oxide or magnesium oxide, and optionally calcium oxide obtainable by mixing an acidic aqueous chitosan solution containing 2 to 40% by weight of chitosan with hydroxyapatite, and zinc oxide or magnesium oxide, and optionally calcium oxide.

The acidic aqueous solution of chitosan used in the present invention contains 2 to 40 % by weight, preferably 3 to 30 % by weight of chitosan. If chitosan is dissolved in an amount of less than 2 % by weight, the composition prepared may have unsatisfactory strength. On the other hand, when the concentration of chitosan is higher than 40 % by weight, a composition having excessively high viscosity may be obtained.

Examples of acids used to solubilize chitosan are hydrochloric acid, acetic acid, formic acid, lactic acid, malic acid, citric acid, adipic acid, tartaric acid and the like. Acids forming insoluble salts with chitosan, such as sulfuric acid, nitric acid, phosphoric acid, molybdic acid, fumaric acid, salicylic acid, phthalic acid are unsuitable in the present invention. The amount of the acid used in the present invention may be so selected that chitosan is solubilized, preferably an amount in the range of from about 50 to 100 parts by weight per 100 parts by weight of chitosan. It is also possible to prepare the acidic chitosan solution by solubilizing chitosan with the acid in saline or Ringer's solution.

Hydroxyapatite, zinc oxide and magnesium oxide used in the present invention may be commercially available products in powder form which should not contain any toxic substance for human body. Hydroxyapatite may be used with zinc oxide and/or magnesium oxide in a ratio by weight of 60 to 99 : 1 to 40, preferably 80 to 99 : 1 to 20. The larger the proportion of zinc oxide and/or magnesium oxide, in particular zinc oxide, is, the shorter will be the hardening time of the resulting composition. Zinc oxide and magnesium oxide may be used independently or in the form of a mixture. It is preferred to use zinc oxide exclusively or predominantly among the two oxides in case of the mixture.

If necessary, calcium oxide can also be used in the hardenable composition of the present invention. By using calcium oxide, the amount of zinc oxide and/or magnesium oxide can be reduced with increasing the amount of hydroxyapatite. In such a case, a ratio by weight of hydroxyapatite to zinc oxide and/or magnesium oxide to calcium oxide may be selected depending on, for example,the desired hardening rate of the resulting hardening composition. The ratio is preferably 60 to 99 : 0.5 to 30 : 0.5 to 10, more preferably 80 to 98 : 1 to 15 : 1 to 5. However, the proportion of calcium oxide should be selected in such way that the pH value of the resulting composition should not exceed 8.

A combining ratio by weight of the acidic aqueous chitosan solution with the above-mentioned powder components is usually selected in the range of 100 : 30 to 250 with depending on the concentration of the chitosan solution used and on a desired density of the hardenable composition to be prepared.

The mixing of the acidic chitosan solution with the powder components may be carried out in an usual way by kneading with using a spatula or a kneader.

Since the present hardenable composition has a pH value of 6 to 8 both in the form of paste and in hardened form, the composition is harmless for organisms. In addition, due to the content of hydroxyapatite, the present hardening composition has an excellent affinity to tooth and bone and, therefore, is very useful in dental and orthopedic fields.

The present invention is further illustrated by the following Examples.

### Examples 1 to 7

To a solution of malic acid (1 g) in Ringer's solution (20 g), chitosan (1 g) was added while heating to prepare an acidic aqueous chitosan solution as a sol. To the sol having a pH value of 3.4, hydroxyapatite, zinc oxide and/or calcium oxide in powder form were added in various amounts as shown in the following Table 1 to obtain compositions in the form of neutral paste, which were subjected to determination of pH values and hardening times. After hardening, compressive strength of each composition was determined. The results are also shown in the Table 1.

**Table 1**

| Example No. | Chitosan solution (g) | Hydroxyapatite (g) | Zinc oxide (g) | Calcium oxide (g) | pH | Hardening time (min.) | Compressive strength (kg/cm²) |
|---|---|---|---|---|---|---|---|
| 1 | 1.0 | 0.45 | 0.05 | 0 | 6.1 | 14 | 10.5 |
| 2 | 1.0 | 0.48 | 0.01 | 0.01 | 6.5 | 19 | 7.9 |
| 3 | 1.0 | 0.47 | 0.01 | 0.02 | 7.9 | 3 | 8.7 |
| 4 | 1.0 | 0.47 | 0.02 | 0.01 | 7.0 | 7 | 14.6 |
| 5 | 1.0 | 0.46 | 0.03 | 0.01 | 7.0 | 2 | 21.6 |
| 6 | 1.2 | 0.46 | 0.03 | 0.01 | 6.9 | 4 | 14.3 |
| 7 | 1.5 | 0.46 | 0.03 | 0.01 | 6.3 | 8 | 4.9 |

## Claims

1. A chitosan-containing hardenable composition in the form of a paste having a pH of 6 to 8, further comprising hydroxyapatite, and zinc oxide and/or magnesium oxide, and optionally calcium oxide obtainable by mixing an acidic aqueous chitosan solution containing 2 to 40% by weight of chitosan with hydroxyapatite, and zinc oxide and/or magnesium oxide, and optionally calcium oxide.

2. The composition according to claim 1, wherein the acidic aqueous chitosan solution contains 3 to 30 % by weight of chitosan.

3. The composition according to claim 1, wherein the acidic aqueous chitosan solution is prepared by solubilizing chitosan by an acid.

4. The composition according to claim 3, wherein the acid is selected from the group consisting of hydrochloric acid, acetic acid, formic acid, lactic acid, malic acid, citric acid, adipic acid and tartaric acid.

5. The composition according to claim 3, wherein the acid is used in an amount in the range of from 50 to 100 parts by weight per 100 parts by weight of chitosan.

6. The composition according to claim 1, wherein a ratio by weight of hydroxyapatite to zinc oxide and/or magnesium oxide is 60 to 99 : 1 to 40.

7. The composition according to claim 6, wherein the weight ratio of hydroxyapatite to zinc oxide and/or magnesium oxide is 80 to 99 : 1 to 20.

8. The composition according to claim 1, which further comprises calcium oxide.

9. The composition according to claim 8, wherein a weight ratio of hydroxyapatite to zinc oxide and/or magnesium oxide to calcium oxide is 60 to 99 : 0.5 to 30 : 0.5 to 10.

10. The composition according to claim 9, wherein the weight ratio of hydroxyapatite to zinc oxide and/or magnesium oxide to calcium oxide is 80 to 98 : 1 to 15 : 1 to 5.

11. The composition according to claim 1, wherein a combining ratio by weight of the acidic aqueous chitosan solution with the other components is selected in the range of 100 : 30 to 250.

12. Use of the compositions according to any of claims 1 to 11 as hardenable filling and/or cushioning material in dental and orthopedic fields.

## Patentansprüche

1. Chitosan enthaltende, härtbare Masse in Form einer Paste mit einem pH-Wert von 6 bis 8, zusätzlich umfassend Hydroxyapatit und Zinkoxid und/oder Magnesiumoxid und gegebenenfalls Calciumoxid, erhältlich durch Vermischen einer sauren wäßrigen Chitosanlösung, die 2 bis 40 Gew.-% Chitosan enthält, mit Hydroxyapatit und Zinkoxid und/oder Magnesiumoxid und gegebenenfalls Calciumoxid.

2. Masse nach Anspruch 1, wobei die saure wäßrige Chitosanlösung 3 bis 30 Gew.-% Chitosan enthält.

3. Masse nach Anspruch 1, wobei die saure wäßrige Chitosanlösung durch Lösen von Chitosan mit einer Säure erhalten wird.

4. Masse nach Anspruch 3, wobei die Säure aus Salzsäure, Essigsäure, Ameisensäure, Milchsäure, Äpfelsäure, Citronensäure, Adipinsäure und Weinsäure gewählt ist.

5. Masse nach Anspruch 3, wobei die Säure in einer Menge im Bereich von 50 bis 100 Gew.-Teilen pro 100 Gew.-Teile Chitosan verwendet wird.

6. Masse nach Anspruch 1, wobei das Gewichtsverhältnis von Hydroxyapatit zu Zinkoxid und/oder Magnesiumoxid 60 bis 99 : 1 bis 40 beträgt.

7. Masse nach Anspruch 6, wobei das Gewichtsverhältnis von Hydroxyapatit zu Zinkoxid und/oder Magnesiumoxid 80 bis 99 : 1 bis 20 beträgt.

8. Masse nach Anspruch 1, zusätzlich Calciumoxid umfassend.

9. Masse nach Anspruch 8, wobei das Gewichtsverhältnis von Hydroxyapatit zu Zinkoxid und/oder Magnesiumoxid zu Calciumoxid 60 bis 99 : 0,5 bis 30 : 0,5 bis 10 beträgt.

10. Masse nach Anspruch 9, wobei das Gewichtsverhältnis von Hydroxyapatit zu Zinkoxid und/oder Magnesiumoxid zu Calciumoxid 80 bis 98 : 1 bis 15 : 1 bis 5 beträgt.

11. Masse nach Anspruch 1, wobei das Gewichtsverhältnis bei der Vereinigung der sauren wäßrigen Chitosanlösung mit den anderen Bestandteilen im Bereich von 100 : 30 bis 250 liegt.

12. Verwendung der Massen nach einem der Ansprüche 1 bis 11 als härtbares Füll- und/oder Einbettungsmaterial auf dentalen und orthopädischen Gebieten.

## Revendications

1. Composition durcissable contenant du chitosan, sous la forme d'une pâte présentant un pH de 6 à 8, comprenant en outre de l'hydroxyapatite et de l'oxyde de zinc et/ou de l'oxyde de magnésium, et facultativement de l'oxyde de calcium, pouvant être obtenue en mélangeant une solution aqueuse acide de chitosan contenant de 2 à 40 % en poids de chitosan, avec l'hydroxyapatite et l'oxyde de zinc et/ou l'oxyde de magnésium, et facultativement l'oxyde de calcium.

2. Composition selon la revendication 1, caractérisée en ce que la solution aqueuse acide de chitosan contient de 3 à 30 % en poids de chitosan.

3. Composition selon la revendication 1, caractérisée en ce que la solution aqueuse acide de chitosan est préparée en solubilisant le chitosan par un acide.

4. Composition selon la revendication 3, caractérisée en ce que l'acide est choisi dans le groupe comprenant l'acide chlorhydrique, l'acide acétique, l'acide formique, l'acide lactique, l'acide malique, l'acide citrique, l'acide adipique et l'acide tartarique.

5. Composition selon la revendication 3, caractérisée en ce que l'acide est utilisé dans une proportion se situant dans la plage d'environ 50 à 100 parties en poids pour 100 parties en poids de chitosan.

6. Composition selon la revendication 1, caractérisée en ce que le rapport de poids de l'hydroxyapatite à l'oxyde de zinc et/ou l'oxyde de magnésium, est de 60 à 99 : 1 à 40.

7. Composition selon la revendication 6, caractérisée en ce que le rapport de poids de l'hydroxyapatite à l'oxyde de zinc et/ou l'oxyde de magnésium, est de 80 à 99: 1 à 20.

8. Composition selon la revendication 1, caractérisée en ce qu'elle comprend en outre de l'oxyde de calcium.

9. Composition selon la revendication 8, caractérisée en ce que le rapport de poids de l'hydroxyapatite à l'oxyde de zinc et/ou oxyde de magnésium à l'oxyde de calcium, est de 60 à 99 : 0,5 à 30 : 0,5 à 10.

10. Composition selon la revendication 9, caractérisée en ce que le rapport de poids de l'hydroxyapatite à l'oxyde de zinc et/ou oxyde de magnésium à l'oxyde de calcium, est de 80 à 98 : 1 à 15 : 1 à 5.

11. Composition selon la revendication 1, caractérisée en ce que le rapport de combinaison en poids de la solution aqueuse acide de chitosan, avec les autres composants, est choisi dans la plage de 100 : 30 à 250.

12. Utilisation des compositions selon l'une quelconque des revendications 1 à 11, comme matériau de remplissage et/ou d'amortissement durcissable dans les domaines dentaires et orthopédiques.
